# EUROPEAN PATENT SPECIFICATION

(11) **EP 1 377 548 B1**
(45) Date of publication and mention of the grant of the patent: **18.02.2009**
(21) Application number: 02701456.2
(22) Date of filing: 08.03.2002
(51) Int. Cl.: C07D 209/08

(54) **PROCESS FOR NITRATING PHOTOCLEAVABLE COMPOUNDS**
VERFAHREN ZUR NITRIERUNG VON PHOTOSPALTBAREN VERBINDUNGEN
PROCEDE PERMETTANT DE NITRER DES COMPOSES PHOTOCLIVABLES

(30) Priority: 11.04.2001 GB 0109093
(43) Date of publication of application: 07.01.2004
(73) Proprietor: Medical Research Council, 20 Park Crescent London W1B 1AL (GB)
(72) Inventor: CORRIE, John Edgar Thomas, Hertfordshire SG3 6DG (GB); PAPAGEORGIOU, George, Hertfor dshire EN4 9AN (GB)
(74) Representative: Kiddle, Simon John
(86) International application number: PCT/GB2002/000971
(87) International publication number: WO 2002/083639

(56) References cited:
- WO-A-00/55133
- DE-A- 3 322 816
- GEORGE PAPAGEORGIOU ET AL.: "Effects of aromatic substituents on the photocleavage of 1-acyl-7-nitroindolines" TETRAHEDRON., vol. 56, - 6 October 2000 (2000-10-06) pages 8197-8205, XP002203072 ELSEVIER SCIENCE PUBLISHERS, AMSTERDAM., NL ISSN: 0040-4020 cited in the application
- JOSEPH P. ADAMS ET AL.: "Nitro and related compounds" JOURNAL OF THE CHEMICAL SOCIETY, PERKIN TRANSACTIONS 1., - 2000 pages 3695-3705, XP002203073 CHEMICAL SOCIETY. LETCHWORTH., GB ISSN: 1472-7781 cited in the application

## Description

### Field of Invention

The present invention relates to a process for producing photocleavable compounds capable of releasing effector moieties on irradiation with light, and in particular to a process of nitrating indolines.

### Background of Invention

Photocleavable (caged) reagents that release biologically active compounds rapidly upon flash irradiation with near-UV light are potentially valuable tools for study of biological processes. However, reagents that can photorelease effectors such as neuroactive amino acids rapidly and efficiently in aqueous solution have proved elusive. Thus, while there are reports in the prior art of approaches to the problem based on a wide range of different photolabile protecting groups, overall these have met with limited success.

In WO 00/55133, a family of photolabile 1-acyl-7-nitroindolines are disclosed which are capable of rapidly and stoichiometrically releasing effectors, such as neuroactive amino acids, peptides and metal ion chelators, where the acyl linkage between the effector and the nitroindoline is broken upon photolysis as exmplified in Scheme 1. 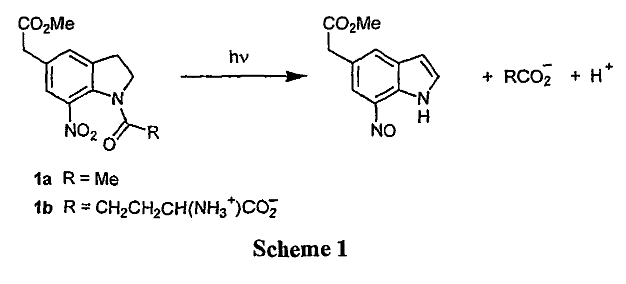 Among the best of the compounds disclosed in this application are compounds having a methoxy group on the 4-position of the heterocyclic ring. Compounds of structure **2** were shown to be ∼3-fold more photosensitive than other useful compounds disclosed in this application such as compounds **1**.² These quantitative comparisons of photolysis efficiency were made with the 1-acetyl compounds **1a** and **2a** and the advantage was maintained for the corresponding L-glutamate derivative **2b.**³

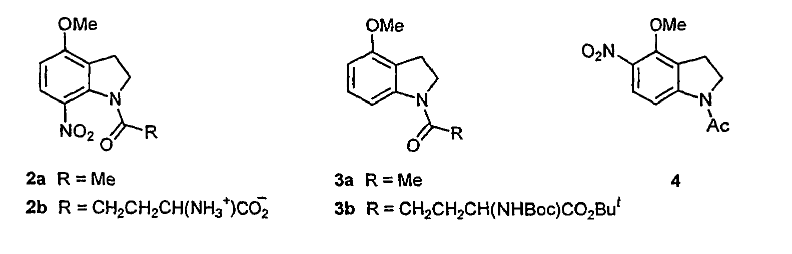

Despite the significant improvement in photosensitivity of **2b** and related compounds, the process for making these compounds suffers from the disadvantage of a poor yield for introduction of the nitro group. In this step, the precursor **3b** was treated with sodium nitrate in TFA solution² and the reaction product was a complex mixture of products, from which the 7-nitro isomer **2b** was isolated by reversed phase HPLC in ∼4% yield. The corresponding 5-nitro isomer was obtained in similar yield and the same 1:1 ratio of 5- and 7-nitro isomers was obtained for the 1-acetyl compound **3a,** where isomers **2a** and **4** could be easily separated by silica gel chromatography. Although the detailed photolysis mechanism is not known, oxygen transfer takes place from the 7-nitro group to the adjacent 1-acyl group,¹ so the 5-isomer is not useful. The previous nitration conditions result in concomitant loss of the protecting groups on the amino acid moiety in **3b,** so separation options were limited to methods appropriate to the zwitterion products.

### Summary of the Invention

Broadly, the present invention relates to an improved process for nitrating photocleavable compounds comprising a photocleavable group linked to an effector moiety which are capable of releasing the effector moiety on irradiation with light or a group is capable of linkage to an effector moiety. In particular, the present invention relates to a process of nitrating precursor compounds having a sensitive substituent at the 4-position, such as an alkoxy group. The process can also be used where the effector is initially linked to the precursor compound, e.g. linked via an acyl linkage to the nitrogen atom at the one position on the heterocyclic ring. This is particularly useful as the prior art nitration methods had the disadvantage of removing protecting groups of present on effector moieties such as amino acids.

The present invention employs copper (II) nitrate and acetic anhydride to nitrate substituted indolines at the 7-position. This reaction which proceeds in good yield and selectivity and can be carried out in the presence of the functional groups at the 4-position and/or the effector moiety which is typically linked via an acyl linkage to the heterocyclic nitrogen at the 1-position. These nitration conditions have the 2-fold advantage of a much more favourable isomeric ratio of products and leaving the protecting groups intact, thereby giving additional scope for isomer separation. In a preferred embodiment, the present invention employs clay supported copper (II) nitrate ("claycop") and acetic anhydride which provides improved yield and isomeric ratio as compared to the use of homogeneous, soluble copper (II) nitrate.

Accordingly, in a first aspect, the present invention provides a process for producing a 7-nitroindoline, the process comprising reacting copper (II) nitrate and acetic anhydride and a compound represented by the formula: 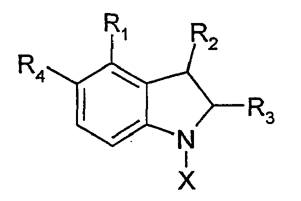 wherein:
- R₁: is an alkoxy or substituted alkoxy group;
- R₂ and R₃: are independently selected from: hydrogen; alkyl or substituted alkyl; or R₂ and R₃ together are cycloalkyl;
- R₄: is hydrogen; alkyl or substituted alkyl; phenyl or substituted phenyl; (CH₂)ₙY; or (CH₂)ₘO(CH₂)ₙY; m and n are independently between 1 and 10; Y is selected from hydrogen, CO₂H or salts thereof, OPO₃²⁻ or salts thereof, or CO₂R₅ , wherein
- R₅: is an alkyl or substituted alkyl group; and
X is an effector moiety linked to the nitrogen atom at the 1-position of the indoline ring via an acyl linkage, or is a group which capable of linkage to an effector moiety;
to produce the 7-nitroindoline.

In the above definition, preferably the alkyl or substituted alkyl groups are C₁₋₁₀ and the alkoxy group is represented by O(CH₂)ₙ-Y, where n and Y are defined above. Particularly preferred classes of compounds are those in which R₄ is hydrogen and R₁ is methoxy.

Preferably, the process is selective for the 7-nitro isomer, that is other isomers are produced in minor amounts. For example, preferably the reaction yields greater than a 5:1 ratio, and more preferably greater than a 12:1 ratio, favouring the production of the 7-nitro isomer.

The nitration reaction can be carried out before or after the reaction to link the effector moiety to the indoline, depending on the sensitivity of the effector moiety or group for linking to the effector moiety to conditions employed in the synthesis and particularly the nitration reaction.

Conveniently, the reaction is carried out at about room temperature. Preferred solvents for the reaction are halogenated alkanes, such as CCl₄ or CH₂Cl₂, or dichloroethane. The use of halogenated alkanes is particularly preferred as they favour the production of the desired 7-nitro isomer. Ethers such as diethyl ether are also suitable solvents for the reaction, with the results with diethyl ether reported herein producing a similar isomeric ratio of products, but having a slower rate of reaction.

The process may comprise the additional step of separating the 7-nitroindoline and optionally, formulating it for further use, e.g. for delivery.

Optionally, the process may comprise the initial step of linking a carboxylic acid group present on an effector moiety to the nitrogen atom at the 1-position of the indoline so that it is linked via an acyl group.

Embodiments of the present invention will now be described in more detail by way of example.

### Detailed Description

### Effector Moieties

The X group can be an effector moiety or a group capable of being coupled to or converted into an effector moiety. The effector moiety is preferably linked to the indoline via an acyl linkage, which may be derived either from a carboxylate moiety inherently present in the effector or as part of a carbamate or urea linkage between the effector and the indoline, for example:

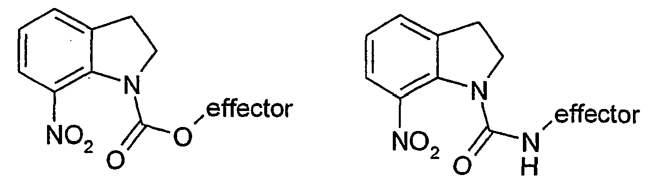

Depending on particular cases, the linkage to a hydroxyl or amino group on the effector may be performed either before or after the nitration reaction according to the present invention. Where the linkage of effector to indoline is carried out post nitration, it can be accomplished as follows:

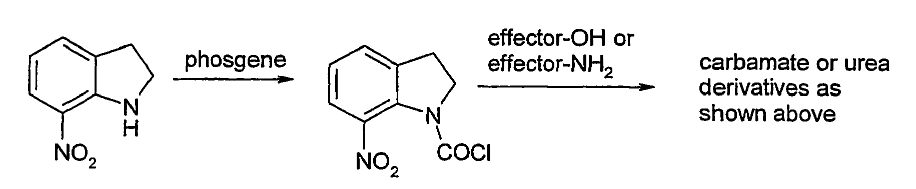

In cases where the effector species is resistant to the nitration conditions, the non-nitrated indoline can be taken through the same steps to create the acyl linkage to the effector moiety.

Examples of effector moieties include labels, drugs, toxins, or carrier or transport molecules. Effectors preferably have a carboxylic acid group which can be coupled to the nitrogen atom at the 1-position on the indoline to form an acyl linked effector.

Techniques for coupling the photocleavable group to both peptidyl and non-peptidyl coupling partners are well known in the art. In preferred embodiments, the effector moiety is a biologically active compound such as an amino acid (either L or D-amino acids), and more particularly neuroactive amino acids such as L-glutamate, GABA and glycine. The procedures described herein can also be readily adapted to linking larger effector groups such as oligopeptides or polypeptides to the photocleavable group. Examples of especially suitable peptides are as follows: thyrotrophin releasing hormone TRH; enkephalins (locally acting endogenous opiates); bradykinin; and angiotensin II. Generally, the methods described herein are applicable to any oligopeptides with non-amidated C-termini.

In further embodiments, the photocleavable group is attached to an effector moiety which is a metal ion chelator thereby providing compounds which on photolysis release the chelator to bind metal ions, e.g. to reduce the metal ion concentration in a system. Thus, by way of example, a photocleavable group can be attached to a carboxylate group containing chelator such as BAPTA (1,2-bis(*o*-aminophenoxy)ethane-*N,N,N',N'*-tetraacetic acid), EDTA (ethylenediaminetetraacetate) or EGTA (ethylene glycol *bis*(β-aminoethyl ether) *N,N,N',N*'-tetraacetic acid). These compounds can then be photolysed to chelate Ca²⁺ or other metal ions. An example of a prior art use of compounds capable of releasing metal ion chelators is provided in Adams et al (12), although the effectiveness of the 5,7-dinitroindoline compounds described in this paper is poor.

The synthesis of photoreleasable compounds including oligo or polypeptide effector moieties can be achieved by linking a terminal amino acid (i.e. the C-terminal amino acid) to the photocleavable group and then using polypeptide chain extension techniques to build up the peptide chain stepwise, or by coupling an oligopeptide or polypeptide to the terminal amino acid linked to the photocleavable group. Standard peptide synthesis techniques could also be adapted by linking to the synthesis resin a suitably substituted alkoxy group at position 4. This would give a resin containing the protecting group and C-terminal residue which could be elaborated and eventually cleaved from the resin by standard techniques.

If the photoreleasable compound is synthesized using the scheme described in the examples below, it is preferred that the effector moiety is stable to the nitration reaction carried out after it is attached to the nitrogen of the indole ring. In the case of amino acids, this means that the use of amino acids other than tryptophan, tyrosine, cysteine or methionine is preferred.

In other aspects, the present invention provides precursor compounds in which the effector moiety has not been linked to the photocleavable group (e.g. X is H, COY as defined above, or COC1) and/or in which the nitration reaction has not been carried out (e.g. the substituent at the 7-position is hydrogen).

### Compositions

The photoreleasable compounds described above can be formulated as compositions. These compositions may comprise, in addition to one or more of the compounds, a pharmaceutically acceptable excipient, carrier, buffer, stabiliser or other materials well known to those skilled in the art. Such materials should be non-toxic and should not interfere with the efficacy of the active ingredient. The precise nature of the carrier or other material may depend on the route of administration, e.g. oral, intravenous, cutaneous or subcutaneous, nasal, intramuscular, or intraperitoneal routes.

In some embodiments, the compounds can be formulated as pharmaceutical compositions for oral administration may be in tablet, capsule, powder or liquid form. A tablet may include a solid carrier such as gelatin or an adjuvant. Liquid pharmaceutical compositions generally include a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included.

For intravenous, cutaneous or subcutaneous injection, or injection at the site of affliction, the photoreleasable compound can be in the form of a parenterally acceptable aqueous solution which is pyrogen-free and has suitable pH, isotonicity and stability. Those of relevant skill in the art are well able to prepare suitable solutions using, for example, isotonic vehicles such as sodium chloride injection, Ringer's injection, lactated Ringer's injection. Preservatives, stabilisers, buffers, antioxidants and/or other additives may be included, as required.

After administration, the photoreleasable compound can be activated to release the effector moiety, conveniently by exposure to a flash of UV light. Preferably, the photoreleasable compound according to the present invention is given to an individual in a "prophylactically effective amount" or a "therapeutically effective amount" (as the case may be, although prophylaxis may be considered therapy), this being sufficient to show benefit to the individual. Typically, this will be to cause a therapeutically useful effect in the patient. The actual amount of the compounds administered, and rate and time-course of administration, will depend on the nature and severity of the condition being treated. Prescription of treatment, e.g. decisions on dosage etc, is within the responsibility of general practitioners and other medical doctors, and typically takes account of the disorder to be treated, the condition of the individual patient, the site of delivery, the method of administration, the data on the efficiency and kinetics of release of the effector moiety and other factors known to practitioners. Examples of the techniques and protocols mentioned above can be found in Remington's Pharmaceutical Sciences, 16th edition, Oslo, A. (ed), 1980.

### Discussion

Exploratory nitrations were carried out on 1-acetyl-4-methoxyindoline **3a**. We showed previously² that **3a** underwent exclusive Friedel-Crafts acetylation at C-7 and hence predicted that nitrosation would also occur at the desired 7-position. Greater regiospecificity than for nitration was expected, as the nitrosonium ion is many orders of magnitude less reactive than the nitronium ion:⁴ a detailed study of nitrosation reactions of electron-rich aromatic compounds with nitrosonium tetrafluoroborate has been reported.⁵ In the event, we were able to reproduce the reported⁵ high-yielding nitrosation of anisole with excess NO⁺BF₄⁻ in dry acetonitrile, but similar treatment of **3a** gave a mixture that still contained ∼50% starting material, together with the *nitrated* isomers **2a** and **4**. The 5-nitro isomer **4** was the more abundant of the two (ratio ∼2:1). Nitration of other substrates under these conditions has been observed when oxygen was present⁶ but the successful control experiment on anisole suggests this was not the case here. While the result is interesting, we chose instead to examine alternative methods for selective 7-nitration.

Among the wide range of nitration reagents,⁷ the clay-supported copper(II) nitrate (claycop) developed by Laszlo and Cornélis⁸ that effects nitration in the presence of acetic anhydride appeared particularly suitable, as the reaction proceeds under conditions unlikely to perturb the tert-butyl protecting groups of **3b**. Furthermore, nitration of acetanilide proceeds with extremely high *ortho*-selectivity.^{8b} We hoped that similar selectivity might obtain for our *N*-acylated substrates **3**.

In either CCl₄ or CH₂Cl₂ as solvent, **3a** was consumed in 4 h and cleanly yielded a mixture of **2a** and **4** in a 5:1 ratio, i.e. strongly favouring the 7-nitro isomer. In diethyl ether as solvent⁹ the reaction was much more sluggish: only ∼20% of **3a** reacted in 4 h and there was no benefit to the product ratio. Gratifyingly, the same reaction on **3b** in CCl₄ overnight cleanly gave the nitrated compounds **5a** and **6**, with the desired isomer **6** as the dominant product (∼1:12 ratio) and the protecting groups intact. Although **2a** and **4** were readily separated by silica gel chromatography, isomers **5a** and **6** were less easily resolved. However, pure **6** was obtained by fractional crystallisation of the mixed isomers, followed by TFA treatment and simple desalting by preparative HPLC to obtain **2b.** The **5a**+**6** mixture could alternatively be treated with TFA to remove the protecting groups and **2b** isolated from the resulting mixture by the 2-stage reversed phase HPLC procedure previously reported.² However, the former procedure was more convenient and the overall yield of **2b** from **3b** was ∼40%.

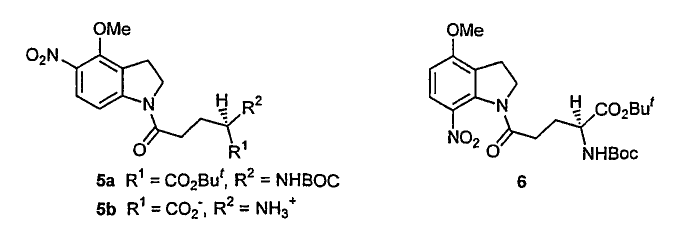

The claycop nitration provides a satisfactory solution to the problem but we also evaluated homogeneous nitration with copper(II) nitrate-acetic anhydride to assess the effect of the clay matrix. Under typical conditions¹⁰ **3a** was completely transformed and the nitrated isomers **2a** and **4** were obtained in 3:2 ratio, i.e. 40% 5-nitro compared to 17% with claycop. Thus the claycop procedure is more favourable and convenient. These results greatly facilitate access to **2b** and related compounds: further applications will be reported elsewhere.

### Experimental

General details were as previously described.² Claycop was prepared with a 2:3 (w/w) ratio of Cu(NO₃)₂ and montmorillonite K10 as described.^{8a} Compounds **1**-**4** were available from previous work.^{1,2} Only the experimental data for the successful claycop nitration of **3b** and subsequent isolation of **2b** are reported here.

**Claycop nitration of 1-{*S*-[4-*t*-butoxycarbonyl)-4-(*t-*butoxycarbonylamino)]-butanoyl-4-methoxyindoline.** Compound **3b** (2.17 g, 5 mmol) was added at room temp to a suspension of claycop (3.2 g) in CCl₄ (20 mL) and acetic anhydride (10 mL) and the mixture was stirred overnight. The solid was filtered and washed with EtOAc and the filtrate was washed with saturated aq. NaHCO₃ and brine, dried and evaporated. Flash chromatography [EtOAc-petroleum ether 45:55] gave two fractions. The first gave a pale yellow gum (322 mg) which was discarded. The second fraction gave a pale solid (1.61 g) that contained a mixture of 5- and 7-nitro isomers **5a** and **6.** Fractional crystallisation (CHCl₃-petroleum ether) gave 1-(*S*-[4-*t*-butoxycarbonyl)-4-(*t-*butoxycarbonylamino)]butanoyl}-4-methoxy-7-nitroindoline **6** (1.03 g, 43%) as yellow crystals, mp 145-147 °C; (Found: C, 57.70; H, 6.98; N, 8.76. C₂₃H₃₃N₃O₈ requires C, 57.61; H, 6.94; N, 8.76%); ¹H NMR: (CDCl₃) δ 7.74 (1H, d, J 8.8 Hz, 6-H), 6.64 (1H, d, 5-H), 5.15 (1H, d, J 7.2 Hz, NH), 4.17-4.25 (3H, m, CH and H-2), 3.91 (3H, s, OMe), 3.04-3.12 (2H, m, 3-H), 2.50-2.56 and 2.58-2.64 (2H, 2 x m, COCH₂), 2.25-2.30 and 1.96-2.03 (2H, 2 x m, CH₂), 1.46 (9H, s, CMe₃), 1.43 (9H, s, CMe₃). The residue in the mother liquor was recrystallised (EtOAc-petroleum ether) to give 1-{*S*-[4-*t*-butoxycarbonyl)-4-(*t-*butoxycarbonylamino)]butanoyl}-4-methoxy-5-nitroindoline **5a** (57 mg, 2%) as yellow crystals, mp 102-103 °C; (Found: C, 57.41; H, 7.12; N, 8.12. C₂₃H₃₃N₃O₈ + ½ EtOAc requires C, 57.35; H, 7.12; N, 8.03%); ¹H NMR: (CDCl₃) δ 8.02 (1H, d, J 9.0 Hz, 7-H), 7.86 (1H, d, 5-H), 5.17 (1H, br s, NH), 4.17-4.26 (1H, m, CH), 4.16 (2H, t, J 8.2, H-2), 3.92 (3H, s, OMe), 3.26 (2H, t, 3-H), 2.46-2.66 (2H, m, COCH₂), 2.26-2.34 and 1.96-2.04 (2H, m, CH₂), 1.48 (9H, s, CMe₃) and 1.42 (9H, s, CMe₃). Signals from the solvent of crystallisation were present at δ 4.12, 2.04 and 1.26.

**1-[*S*-(4-Amino-4-carboxybutanoyl)]-4-methoxy-7-nitroindoline 2b**. A solution of **6** (479 mg, 1 mmol) in TFA (15 mL) was stirred at room temp for 1 h and concentrated *in vacuo*. The residue was dissolved in water (60 mL) and adjusted to pH 7.0 with 1 M aq. NaOH. The solution was washed with ether and analysed by reversed phase HPLC (mobile phase 25 mM Na phosphate, pH 6.0 + 25% MeCN (v/v); 1.5 mL/min), *t*_{R} 4.2 min. The solution was lyophilised, redissolved in 25 mM Na phosphate, pH 6.0 (60 mL) and pumped onto a preparative reversed phase HPLC column (details as described²). The column was first washed with water for 2 h, then the product was eluted with water + 20% MeCN (v/v; all flow rates 2.5 mL/min). Fractions containing the product were analysed, combined and quantified (UV spectroscopy) to give a solution of **2b** (867 µmol, 87%). The solution was passed through a 0.2 µm filter, lyophilised and stored at -20 °C as a pale yellow powder. Fractions from the very end of the eluted peak contained a trace amount of **5b**, *t*_{R} 5.6 min, and were discarded without significant loss of **2b**.

### References:

The following references are all expressly incorporated by reference.
1. Papageorgiou, G., Ogden, D.C., Barth A. and Corrie, J.E.T. J. Am. Chem. Soc. 1999, 121, 6503.
2. Papageorgiou, G. and Corrie, J.E.T. Tetrahedron 2000, 56, 8197.
3. Canepari, M., Nelson, L., Papageorgiou, G., Corrie, J.E.T. and Ogden, D. Biophys. J. 2001, 80, 107a.
4. March, J. "Advanced Organic Chemistry," 4th Edition, Wiley, New York, 1992, p. 525.
5. Bosch, E. and Kochi, J.K. J. Org. Chem. 1994, 59, 5573.
6. Kim, E.K. and Kochi, J.K. J. Org. Chem. 1989, 54, 1692.
7. (a) Olah, G.A., Malhotra, R. and Narang, S.C. "Nitration: Methods and Mechanisms," VCH, New York, 1989, pp. 9-116; (b) Adams, J.P. and Paterson, J.R. J. Chem. Soc., Perkin Trans. 1 2000, 3695.
8. (a) Laszlo, P. and Cornélis, A. Aldrichim. Acta 1988, 21, 97; (b) Gigante, B., Prazeres, A.O., Marcelo-Curto, M.J., Cornélis, A. and Laszlo, P. J. Org. Chem. 1995, 60, 3445.
9. Dwyer, C.L. and Holzapfel, C.W. Tetrahedron 1998, 54, 7843.
10. Yamato, T., Kamimura, H. and Tsuzuki, H. Can. J. Chem. 1998, 76, 997.
11. WO 00/55133 (Medical Research Council, Corrie and Papageorgiou).
12. Adams et al, J. Am. Chem. Soc., 1989, 111, 7957.

## Claims

1. A process for producing a 7-nitroindoline, the process comprising reacting a substituted indoline with copper (II) nitrate and acetic anhydride to produce the 7-nitroindoline.

2. The process of claim 1, wherein the substituted indoline is represented by the formula: 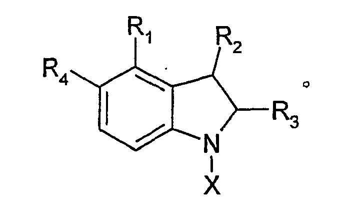 wherein:
R₁ is an alkoxy or substituted alkoxy group;
R₂ and R₃ are independently selected from: hydrogen; alkyl or substituted alkyl; or R₂ and R₃ together are cycloalkyl;
R₄ is hydrogen; alkyl or substituted alkyl; phenyl or substituted phenyl; (CH₂)ₙY; or (CH₂)ₘO(CH₂)ₙY;
m and n are independently between 1 and 10;
Y is selected from hydrogen, CO₂H or salts thereof, OPO₃²⁻ or salts thereof, or CO₂R₅ , wherein R₅ is an alkyl or substituted alkyl group; and
X is an effector moiety linked to the nitrogen atom at the 1-position of the indoline ring via an acyl linkage, or is a group which capable of linkage to an effector moiety.

3. The process of claim 1 or claim 2, wherein the copper (II) nitrate is clay supported ("claycop").

4. The process of claim 1 or claim 2, wherein the copper (II) nitrate is soluble.

5. The process of any one of claims 1 to 4, wherein X is a group capable of linkage to an effector moiety and the process comprises the further step of linking the 7-nitroindoline to an effector moiety, forming an acyl linkage between the indoline and the effector.

6. The process of claim 5, wherein X is H, COY or COCl.

7. The process of any one of claims 1 to 4, wherein X is an effector moiety and the process comprises the initial step of linking the indoline to the effector moiety via an acyl linkage.

8. The process of any one of claims 2 to 7, wherein the acyl linkage is a carbamate or urea linkage.

9. The process of any one of claims 2 to 8, wherein the alkyl or substituted alkyl groups are C₁₋₁₀

10. The process of any one of claims 2 to 9, wherein the alkoxy group is represented by O(CH₂)ₙ-Y.

11. The process of any one of claims 2 to 10, wherein R₄ is hydrogen and R₁ is methoxy.

12. The process of any one of the preceding claims, wherein the process yields greater than a 5:1 ratio, favouring the production of the 7-nitro isomer.

13. The process of any one of the preceding claims, wherein the reaction is carried out at about room temperature.

14. The process of any one of the preceding claims, wherein the reaction is carried out in a solvent which is a halogenated alkane or dichloroethane.

15. The process of claim 14, wherein the halogenated alkane is CCl₄ or CH₂Cl₂.

16. The process of any one of the preceding claims, further comprising the step of separating the 7-nitroindoline.

17. The process of claim 16, further comprising formulating the compound for use.

18. The process of claim 16 or claim 17, further comprising exposing the compound to light to release the effector moiety.

19. The process of any one of claims 2 to 18, wherein the effector moiety is a biologically active compound.

20. The process of any one of claims 2 to 19, wherein the effector moiety is an amino acid, a neurotransmitter, an oligopeptide, a polypeptide or a metal ion chelator.

21. The process of claim 20, wherein the neurotransmitter is L-glutamate, GABA or glycine.

22. The process of claim 20, wherein the polypeptide is thyrotrophin releasing hormone (TRH), an enkephalin, bradykinin or angiotensin II.

23. The process of claim 20, wherein the metal ion chelator is a carboxylate group containing metal ion chelator.

24. The process of claim 23, wherein the metal ion chelator is BAPTA (1,2-bis(o-aminophenoxy)ethane-*N,N,N',N'*-tetraacetic acid), EDTA (ethylenediaminetetraacetate) or EGTA (ethylene glycol *bis*(β-aminoethyl ether) *N,N,N',N'*-tetraacetic acid).

## Patentansprüche

1. Verfahren zur Produktion eines 7-Nitroindolins, wobei das Verfahren das Umsetzen eines substituierten Indolins mit Kupfer(II)-Nitrat und Essigsäureanhydrid umfasst, um das 7-Nitroindolin zu erzeugen.

2. Verfahren nach Anspruch 1, worin das substituierte Indolin durch folgende Formel dargestellt ist: 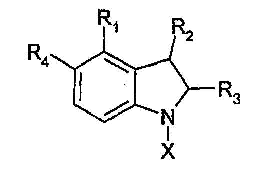 worin:
R₁ eine Alkoxy- oder eine substituierte Alkoxy-Gruppe ist;
R₂ und R₃ unabhängig voneinander aus Folgendem ausgewählt sind: Wasserstoff; Alkyl oder substituiertem Alkyl;
oder R₂ und R₃ zusammen Cycloalkyl sind;
R₄ Wasserstoff; Alkyl oder substituiertes Alkyl; Phenyl oder substituiertes Phenyl; (CH₂)ₙY; oder (CH₂)ₘO(CH₂)ₙY ist;
m und n unabhängig voneinander zwischen 1 und 10 liegen;
Y aus Wasserstoff, CO₂H oder Salzen davon, OPO₃²⁻ oder Salzen davon und CO₂R₅ ausgewählt ist, worin R₅ eine Alkyl- oder eine substituierte Alkyl-Gruppe ist; und
X eine Effektorgruppierung ist, die über eine Acylbindung an das Stickstoffatom an Position 1 des Indolin-Rings gebunden ist, oder eine Gruppe ist, die zur Bindung an eine Effektorgruppierung in der Lage ist.

3. Verfahren nach Anspruch 1 oder Anspruch 2, worin das Kupfer(II)-Nitrat auf einem Tonträger vorliegt ("Claycop").

4. Verfahren nach Anspruch 1 oder Anspruch 2, worin das Kupfer(II)-Nitrat löslich ist.

5. Verfahren nach einem der Ansprüche 1 bis 4, worin X eine Gruppe ist, die in der Lage ist, an eine Effektorgruppierung zu binden, und das Verfahren weiters den Schritt des Bindens des 7-Nitroindolins an eine Effektorgruppierung umfasst, wodurch eine Acylbindung zwischen dem Indolin und dem Effektor gebildet wird.

6. Verfahren nach Anspruch 5, worin X H, COY oder COCl ist.

7. Verfahren nach einem der Ansprüche 1 bis 4, worin X eine Effektorgruppierung ist und das Verfahren den anfänglichen Schritt des Bindens des Indolins an die Effektorgruppierung über eine Acylbindung umfasst.

8. Verfahren nach einem der Ansprüche 2 bis 7, worin die Acylbindung eine Carbamat- oder Harnstoffbindung ist.

9. Verfahren nach einem der Ansprüche 2 bis 8, worin die Alkyl- oder substituierten Alkyl-Gruppen C₁₋₁₀ sind.

10. Verfahren nach einem der Ansprüche 2 bis 9, worin die Alkoxy-Gruppe durch O(CH₂)ₙ-Y dargestellt ist.

11. Verfahren nach einem der Ansprüche 2 bis 10, worin R₄ Wasserstoff ist und R₁ Methoxy ist.

12. Verfahren nach einem der vorangegangenen Ansprüche, worin das Verfahren ein Verhältnis von mehr als 5:1 ergibt, so dass die Produktion des 7-Nitro-Isomers gefördert wird.

13. Verfahren nach einem der vorangegangenen Ansprüche, worin die Reaktion etwa bei Raumtemperatur durchgeführt wird.

14. Verfahren nach einem der vorangegangenen Ansprüche, worin die Reaktion in einem Lösungsmittel durchgeführt wird, das ein halogeniertes Alkan oder Dichlorethan ist.

15. Verfahren nach Anspruch 14, worin das halogenierte Alkan CCl₄ oder CH₂Cl₂ ist.

16. Verfahren nach einem der vorangegangenen Ansprüche, das weiters den Schritt des Abtrennens des 7-Nitroindolins umfasst.

17. Verfahren nach Anspruch 16, das weiters das Formulieren der Verbindung für deren Verwendung umfasst.

18. Verfahren nach Anspruch 16 oder Anspruch 17, das weiters das Aussetzen der Verbindung gegenüber Licht zur Freisetzung der Effektorgruppierung umfasst.

19. Verfahren nach einem der Ansprüche 2 bis 18, worin die Effektorgruppierung eine biologisch aktive Verbindung ist.

20. Verfahren nach einem der Ansprüche 2 bis 19, worin die Effektorgruppierung eine Aminosäure, ein Neurotransmitter, ein Oligopeptid, ein Polypeptid oder ein Metallionenchelatbildner ist.

21. Verfahren nach Anspruch 20, worin der Neurotransmitter L-Glutamat, GABA oder Glycin ist.

22. Verfahren nach Anspruch 20, worin das Polypeptid Thyrotropin freisetzendes Hormon (TRH), ein Enkephalin, Bradykinin oder Angiotensin-II ist.

23. Verfahren nach Anspruch 20, worin der Metallionenchelatbildner ein eine Carboxylatgruppe enthaltender Metallionenchelatbildner ist.

24. Verfahren nach Anspruch 23, worin der Metallionenchelatbildner BAPTA (1,2-Bis(o-aminophenoxy)ethan-N,N,N',N'-tetraessigsäure), EDTA (Ethylendiamintetraacetat) oder EGTA (Ethylenglykol-bis(β-aminoethylether)-N,N,N',N'-tetraessigsäure) ist.

## Revendications

1. Procédé pour produire une 7-nitroindoline, le procédé comprenant la mise en réaction d'une indoline substituée avec du nitrate de cuivre (II) et un anhydride acétique pour produire la 7-nitroindoline.

2. Procédé selon la revendication 1, où l'indoline substituée est représentée par la formule: 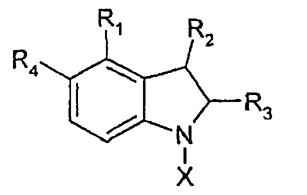 où:
R₁ est un alcoxy ou un groupe alcoxy substitué;
R₂ et R₃ sont indépendamment sélectionnés parmi: hydrogène; alkyle ou alkyle substitué; ou R₂ et R₃ ensemble sont cycloalkyle;
R₄ est hydrogène; alkyle ou alkyle substitué; phényle ou phényle substitué; (CH₂)ₙY; OU (CH₂)ₘO (CH₂)ₙY;
m et n sont indépendamment entre 1 et 10;
Y est sélectionné parmi hydrogène, CO₂H ou des sels de ceux-ci, OPO₃²⁻ ou des sels de ceux-ci ou CO₂R₅, où R₅ est alkyle ou un groupe alkyle substitué; et
X est un fragment effecteur lié à l'atome d'azote à la position-1 du cycle d'indoline par une liaison acyle, ou est un groupe qui est apte à se lier à un fragment effecteur.

3. Procédé selon la revendication 1 ou la revendication 2, où le nitrate de cuivre (II) est de l'argile supporté ("claycop").

4. Procédé selon la revendication 1 ou la revendication 2, où le nitrate de cuivre (II) est soluble.

5. Procédé selon l'une quelconque des revendications 1 à 4, où X est un groupe apte à se lier à un fragment effecteur, et le procédé comprend l'étape ultérieure consistant à lier la 7-nitroindoline à un fragment effecteur, en formant une liaison acyle entre l'indoline et l'effecteur.

6. Procédé selon la revendication 5, où X est H, COY ou COCl.

7. Procédé selon l'une quelconque des revendications 1 à 4, où X est un fragment effecteur, et le procédé comprend l'étape initiale consistant à lier l'indoline au fragment effecteur par une liaison acyle.

8. Procédé selon l'une quelconque des revendications 2 à 7, où la liaison acyle est un carbamate ou une liaison urée.

9. Procédé selon l'une quelconque des revendications 2 à 8, où l'alkyle ou les groupes alkyles substitués sont C₁₋₁₀.

10. Procédé selon l'une quelconque des revendications 2 à 9, où le groupe alcoxy est représenté par O(CH₂)ₙ-Y.

11. Procédé selon l'une quelconque des revendications 2 à 10, où R4 est hydrogène et R₁ est méthoxy.

12. Procédé selon l'une quelconque des revendications précédentes, où le procédé obtient plus qu'un rapport 5:1, favorisant la production du 7-nitro isomère.

13. Procédé selon l'une quelconque des revendications précédentes, où la réaction est exécutée environ à température ambiante.

14. Procédé selon l'une quelconque des revendications précédentes, où la réaction est exécutée dans un solvant qui est un alcane halogéné ou dichloroéthane.

15. Procédé selon la revendication 14, où l'alcane halogéné est CCl₄ ou CH₂Cl₂.

16. Procédé selon l'une quelconque des revendications précédentes, comprenant en outre l'étape consistant à séparer la 7-nitroindoline.

17. Procédé selon la revendication 16, comprenant en outre la formulation du composé pour l'utilisation.

18. Procédé selon la revendication 16 ou la revendication 17, comprenant en outre l'exposition du composé à la lumière pour libérer le fragment effecteur.

19. Procédé selon l'une quelconque des revendications 2 à 18, où le fragment effecteur est un composé biologiquement actif.

20. Procédé selon l'une quelconque des revendications 2 à 19, où le fragment effecteur est un acide aminé, un neurotransmetteur, un oligopeptide, un polypeptide ou un chélateur d'ions métalliques.

21. Procédé selon la revendication 20, où le neurotransmetteur est L-glutamate, GABA ou glycine.

22. Procédé selon la revendication 20, où le polypeptide est une hormone de libération de la thyréostimuline (TRH), une encéphaline, bradykinine ou angiotensine II.

23. Procédé selon la revendication 20, où le chélateur d'ions métalliques est un groupe de carboxylate contenant un chélateur d'ions métalliques.

24. Procédé selon la revendication 23, où le chélateur d'ions métalliques est un BAPTA (acide 1,2-bis(o-aminophénoxy)éthane-*N,N,N',N'*-tétraacétique), EDTA (éthylènediaminetétraacétate) ou EGTA (acide d'éthylène glycol bis(β-aminoéthyl éther) *N,N,N',N'*-tétraacétique.
